# EUROPEAN PATENT APPLICATION

(11) **EP 2 952 572 A1**
(43) Date of publication of application: **09.12.2015**
(21) Application number: 15001601.2
(22) Date of filing: 28.05.2015
(51) Int. Cl.: C12M 3/00, C12M 1/00

(54) **INCUBATOR WITH CONTROLLED ILLUMINATION**

(30) Priority: 03.06.2014 CH 852142014
(71) Applicant: LICONIC AG, 9493 Mauren (LI)
(72) Inventor: Cosmas, Malin, 9493 Mauren (LI)
(74) Representative: Sutter, Kurt

(57) **Abstract**

The incubator comprises a climate controlled chamber (4) with a plurality of containers (5) located therein. Each container (5) can receive a sample plate (8) and is adapted with light sources (14) for exposing the samples to light. The incubator further comprises a handling device (6) for transferring sample plates between the containers (5) and a transfer location positioned outside an automatic door (10). A cooling unit (17) is provided for carrying off excess heat from the light sources (14).

## Description

### Technical Field

The invention relates to an incubator with a climate controlled chamber and light sources arranged in said chamber. The invention also relates to a container to be used in such an incubator.

### Background Art

In various fields of science, in particular in biology and chemistry, there is a need to expose samples to a controlled environment and controlled optical illumination. For example, in optogenetics, biological samples have to be exposed to light of certain spectral properties while being maintained under controlled temperature and humidity.

Typically, such procedures rely on conventional incubators, which have been retrofitted with a suitable light source.

### Disclosure of the Invention

The problem to be solved by the present invention is therefore to provide equipment of this type that allows to efficiently carry out experiments that require controlled optical illumination of the samples.

This problem is solved by the incubator and by the container of the independent claims.

Accordingly, the invention relates to an incubator comprising the following components:
- A chamber: This chamber forms the climate-controlled space of the incubator;
- A climate controller for controlling the climate in said chamber: This controller advantageously controls at least one of the parameters of the climate within the chamber. These parameters e.g. include at least one of temperature, humidity, oxygen content, carbon dioxide content and other gas content within the chamber. In a particularly advantageous embodiment, the climate controller controls at least the temperature and humidity within the chamber.
- A plurality of containers arranged in said chamber, wherein each container encloses an interior space for receiving at least one sample and comprises at least one light source arranged to illuminate said interior space.

Such an incubator allows to individually control the illumination in each container while the climate can be controlled globally for all containers.

The incubator can comprise an illumination controller connected to the light sources of the containers. The illumination controller is adapted and structured to individually control at least one of a duration, intensity and spectral distribution of the illumination in each of said interior spaces. Hence, the illumination conditions in said containers can differ from each other.

Further, the incubator can comprise at least one cooling assembly for carrying off heat from said containers. This allows to carry off the heat generated by the light sources, thereby allowing to maintain a more stable climate in each container.

This cooling assembly advantageously coupled to a common cooling unit that is structured and adapted to cool a cooling fluid. Further, the incubator comprises a plurality of ducts carrying the cooling fluid between the cooling unit and the containers for cooling each container.

For improved individual temperature control, each container can be equipped with at least one temperature sensor. In that case, the incubator can comprise a number of control loops, implemented in hardware or software, for controlling the temperatures in said containers, thereby maintaining these temperatures at one or more desired temperature values.

In order to efficiently use the space within the chamber of the incubator, several of the containers are arranged therein (horizontally) beside each other and several of the containers are arranged (vertically) above each other. In particular, the incubator can comprise a plurality of storage racks arranged beside each other within said chamber. Each storage rack holds several containers vertically above each other.

The incubator can further comprise a handling device arranged in the climate controlled chamber and an automatic door arranged in a wall of said chamber. The handling device is adapted to transport samples between the door and the containers. This allows to efficiently and securely handle a large number of experiments or experimental steps.

The invention also relates to a container for receiving at least one sample and exposing said sample to controlled illumination, in particular as it can be used in an incubator as described above. This container comprises:
- A housing enclosing an interior space for receiving said at least one sample.
- An opening arranged in a wall of the housing. This opening is used for moving the sample(s) into and from the interior space.
- At least one light source arranged to illuminate said interior space.

In an advantageous embodiment, the at least one light source is mounted to a cooling assembly for carrying off heat generated by the light source(s). Such a cooling assembly can e.g. comprise a channel for carrying a cooling fluid and an adapter for connecting the channel to an external source of cooling fluid, such as the cooling unit described above.

The opening in the container should be small compared to the area of the wall that it is located in, thereby minimizing the amount of light that can escape from the interior space. Advantageously, the area of the opening should be at most 25% of the area of the interior side (i.e. the side facing the interior space) of the wall.

Advantageously, the interior space and the opening of the container are dimensioned to horizontally receive sample holding plates that are microplates according to the ANSI standard. Such plates have a length of 127.76 ± 0.5 mm, a width of 85.48 ± 0.5 mm and a height of 14.35 ± 0.76 mm.

With a sample holding plate arranged in the interior space, the light source(s) should advantageously be arranged above the sample holding plate. In order to obtain a homogeneous illumination, the vertical distance between the light source(s) and the sample holding plate should not be too small, advantageously it should be at least 20 mm.

Also for obtaining a homogeneous illumination, the container advantageously comprises a plurality of light sources, in particular arranged in a repeating pattern.

The light sources advantageously comprise different light sources with peak emission wavelengths differing by at least 100 nm. This allows the control unit of the incubator to vary the illumination spectrum by selectively activating varying individual light sources. For example, a first group of light sources can emit red light, a second group of light sources can emit green light, and a third group of light sources blue light.

Other advantageous embodiments are listed in the dependent claims as well as in the description below.

### Brief Description of the Drawings

The invention will be better understood and objects other than those set forth above will become apparent from the following detailed description thereof. Such description makes reference to the annexed drawings, wherein:
Fig. 1 shows a block diagram of some components of an incubator,
Fig. 2 is a first view of an implementation of the incubator (with chamber walls not drawn),
Fig. 3 is a second view of the incubator,
Fig. 4 shows the carousel with the containers and the handling device in a first view,
Fig. 5 shows the components of Fig. 4 in a second view,
Fig. 6 shows a single container with a sample plate in partially transparent view, and
Fig. 7 shows the container of Fig. 6 with a cooling assembly.

### Modes for Carrying Out the Invention

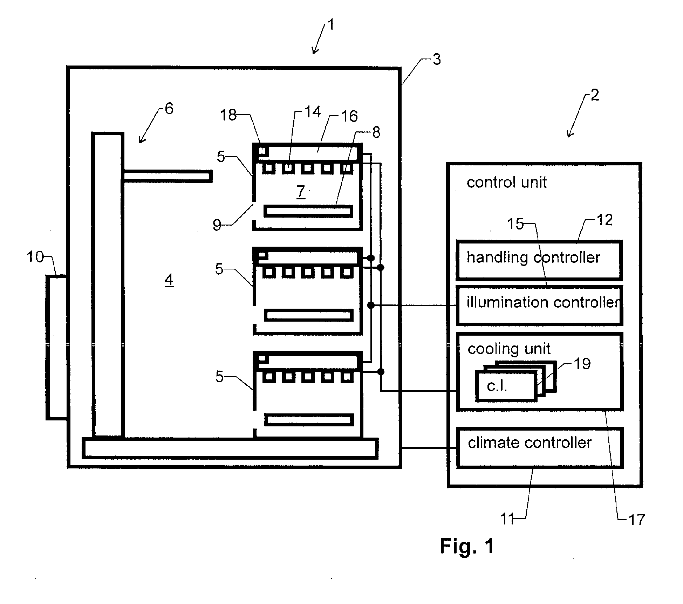

Fig. 1 shows an incubator 1 in schematic view with the components of its control unit 2. Even though in Fig. 1 control unit 2 is, for clarity, illustrated as a box outside the actual incubator housing 3, it is advantageously located within housing 3.

Housing 3 of incubator 1 encloses a climate controlled chamber 4, which typically has insulated walls. Located within climate chamber 4 is a plurality of containers 5 as well as a handling device 6.

Each container 5 encloses an interior space 7 adapted for receiving samples in a storage plate 8. Interior space 7 has an opening 9 in one of its walls. Opening 9 forms a passage for inserting/removing storage plate 8 into/from interior space 7.

From the outside, chamber 4 is accessible through an automatic door 10. Handling device 6 is adapted to move the storage plates 8 between their locations inside the containers 5 and an exchange location arranged outside door 10.

Control unit 2 of the incubator comprises a climate controller 11, which maintains a controlled climate within chamber 4. Advantageously, climate controller 11 comprises a heater or heat pump for controlling the temperature within chamber 4 as well as a humidifier for controlling the humidity therein.

Control unit 2 further comprises a handling controller 12 adapted and structured to operate handling device 6, door 10 as well as a carousel to be described below.

As mentioned above, the present incubator is used to illuminate the samples with light. For this purpose, each container 5 is equipped with a plurality of light sources 14 advantageously arranged at the top of its interior space 7. These light sources can e.g. be LEDs of different colours in order to provide a broader spectral range or to selectively choose a desired spectral range, and they can be arranged in a repeating pattern for generating a light field of good homogeneity.

The operation of the light sources 14 of each container 5 can be individually controlled by an illumination controller 15 of control unit 2. In particular, illumination controller 15 is adapted to operate the light sources 14 of each container 5 according to a user selectable or programmable sequence, by switching individual light sources on or off or by varying their intensity.

The temperature within chamber 4 and in particular within the interior spaces 7 should be controlled accurately. This is particularly true if the humidity levels are to be high because, in that case, temperature gradients give rise to humidity gradients and condensation. Therefore, the containers 5 are advantageously provided with cooling assemblies 16 for carrying off the heat from the light sources 14. The cooling assemblies 16 are operated by a cooling unit 17. For example, cooling unit 17 can comprise a heat pump adapted for cooling a cooling fluid, and a fluid pump for pumping the cooling fluid through the cooling assemblies 16.

Each container 5 can optionally be equipped with a temperature sensor 18, and cooling unit 17 can comprise feedback loop controllers 19 for keeping the temperature measured by temperature sensor 18 at a desired level for each container 5 individually.

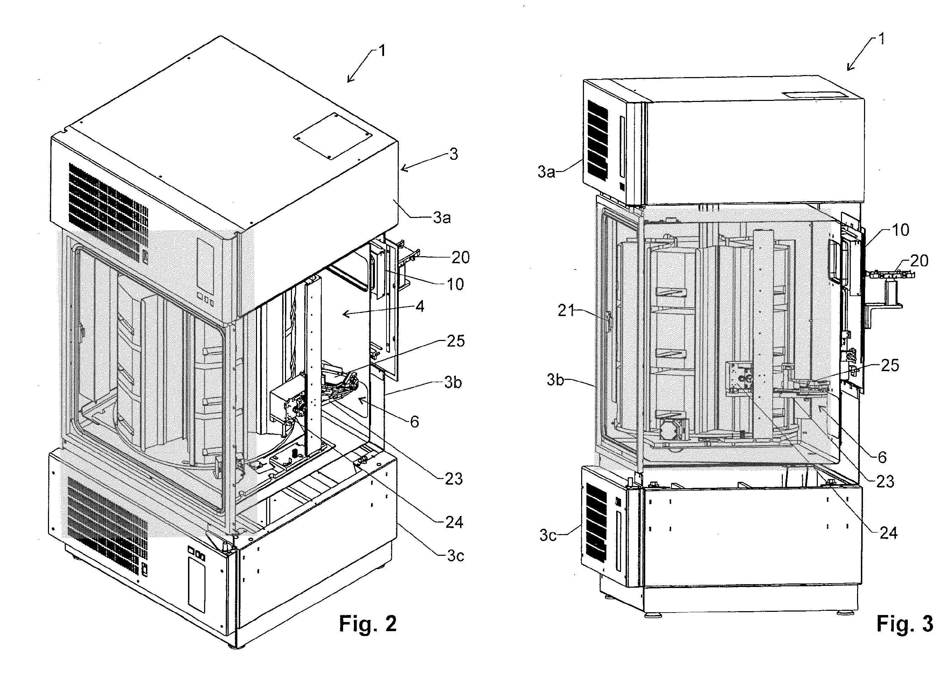

Figs. 2 and 3 show a specific embodiment of the incubator. Its housing 3 comprises a top section 3a, a middle section 3b and a bottom section 3c. Top section 3a can e.g. contain the parts of control unit 2 that are specific to an illuminated incubator, such as illumination controller 15 and cooling unit 17. Middle section 3b contains chamber 4. Bottom section 3c contains the remaining parts of control unit 2.

The automatic door 10 of chamber 4 can best be seen in Fig. 3, with a transfer location just outside door 10 formed by a plate mount 20 affixed to the outside of housing 3.

Opposite to automatic door 10, the incubator comprises a user access door 21, which provides service access to chamber 4, e.g. for cleaning and maintenance.

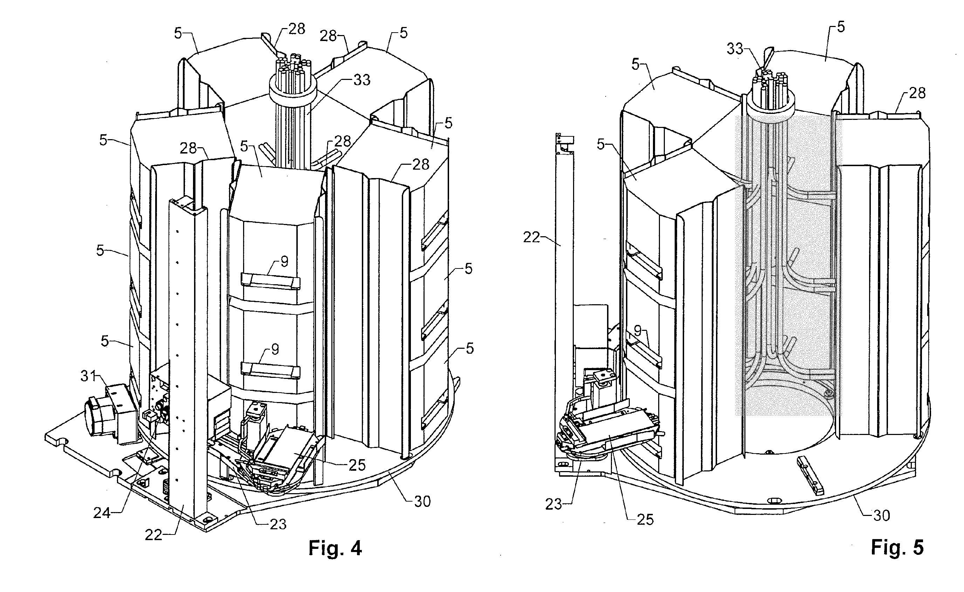

As shown in Figs. 4 and 5, handling device 6 comprises an elevator assembly formed by a vertical guide rail 22, a carriage 23, and an elevator drive 24 for displacing carriage 23 along guide rail 22. Carriage 23 carries a scoop assembly 25, which is pivotal about a vertical axis and extendible horizontally.

The present embodiment of the incubator is equipped with 15 containers 5. They are arranged in three levels, with five containers on each level. The containers 5 are mounted to storage racks 28, with each storage rack 28 holding three containers 5 on top of each other.

The storage racks 28 rest on a carousel 30. A carousel drive 31 is provided for rotating carousel 30 about a vertical, central axis.

By rotating carousel 30 about its vertical axis, the three containers 5 of each storage rack 28 can be brought into the access range of handling device 6, such that its scoop assembly 25 can slide through the outward facing opening 9 of each container 5 in order to deposit or retrieve a storage plate.

By rotating scoop assembly 25 about its vertical axis and by suitably displacing carriage 23 along guide rail 22, scoop assembly 25 can also be aligned with door 10. After opening door 10, scoop assembly 25 can be extended to access plate mount 20 at the transfer location.

Figs. 4 and 5 also show a plurality of cables and ducts 33, which are used for transmitting electrical signals as well as the cooling fluid between top section 3a of housing 3 and the individual containers 5. In order to avoid excessive torsion of the cables and ducts 33, the rotation of carousel 30 is limited to +/- 180° starting from a non-twisted position of the cables and ducts 33.

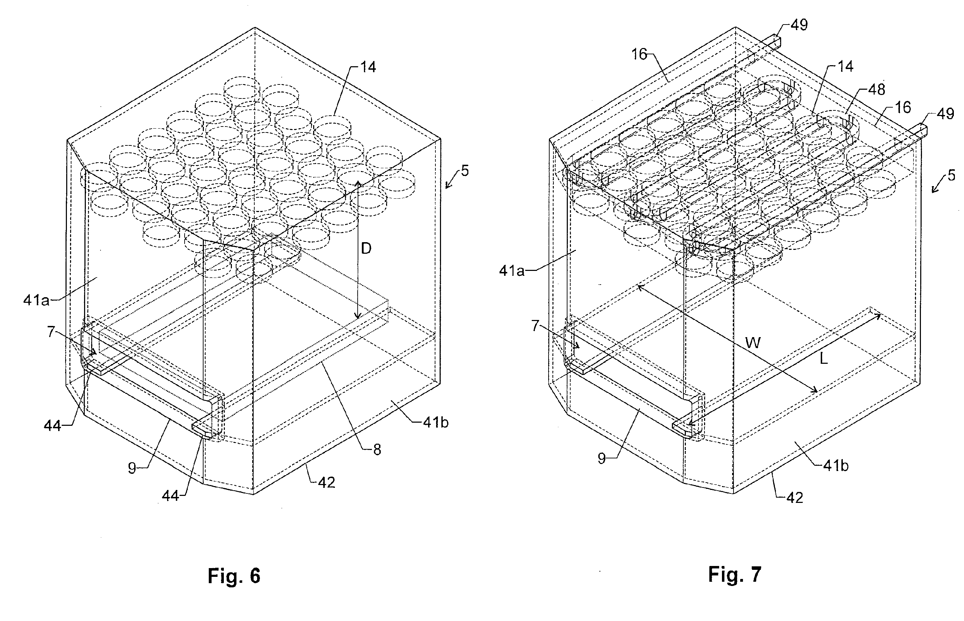

Figs. 6 and 7 show a container 5 in more detail. It comprises a top wall 40, four side walls 41 and a bottom wall 42, which enclose interior space 7. The walls are advantageously of stainless steel.

The interior walls of the container 5 that face interior space 7 should have a high total reflectivity, in particular a reflectivity of at least 80% over the wavelengths of the light sources 14. Such a high reflectivity reduces unnecessary light losses and increases the homogeneity of the light distribution within interior space 7.

Located near the bottom of interior space 7 there are two lateral supports 44 for receiving a sample plate 8. The top sides of the supports 44 are somewhat higher than the bottom side of opening 9 in order to provide a gap for scoop assembly 25 between the bottom side of opening 9 and a bottom side of the sample plate 8.

Fig. 6 shows, in dotted lines, a sample plate 8 at its sample plate location within interior space 7. Advantageously, sample plate 8 forms a plurality of receptacles for receiving a number of samples, as known in the prior art. Typically, the sample plate has a standardized size with the dimensions given above.

The horizontal width W and length L of interior space 7 should be somewhat larger than the width and length of the sample plate. If these dimensions are too close to the dimensions of the sample plate, the light intensity at the edges of the sample plate becomes hard to control. If the interior space 7 is, however, too large as compared to the sample plate, precious space is lost. Advantageously, for the size of sample plates mentioned above, the width W should be between 100 and 130 mm, while the length should be between 140 and 170 mm.

If the distance D between sample plate 8 and the light sources 14 above it is too small, a homogeneous light distribution is hard to achieve. Advantageously, this distance D should be at least 20 mm.

Depending on distance D and the spatial density of the light sources 14, the optical half-intensity radiation angle (i.e. the angle range cantered about the LEDs emission axis over which the intensity varies by less than 50%) should be at least 45°, in particular at least 60°.

Opening 9 should be comparatively small in order to avoid light loss and optical cross talk between neighbouring containers 5. For this reason, opening 9 should have an area of at most 25% of the area of the interior side of the wall 41a that it is located in, but this may depend on the size of the sample plate.

The dimensions of opening 9 should be slightly above the width and height of sample plate 8, or, for transverse insertion of sample plate 8, slightly above it length and height. Hence, for the standard dimensions mentioned above, the width of opening 9 should not exceed 140 mm (for transverse insertion) or 100 mm (for longitudinal insertion of the sample plate), and its height should not exceed 30 mm.

Fig. 7 shows cooling assembly 16 in more detail. It comprises a metal body forming a meandering channel 48 for the cooling fluid and an adapter 49 for connecting channel 48 to the ducts 33 leading to cooling unit 17.

### Notes:

The term "light" or "illumination" as used herein and in the claims is understood to refer to infrared, visible and ultraviolet light, in particular in the wavelength range between 2 µm and 0.2 µm, in particular between 1 µm and 0.3 µm. The light sources used in the containers should therefore advantageously have a peak wavelength somewhere within that range.

As mentioned, each container 5 can be equipped with several light sources 14. The wavelength ranges of the light sources can differ, which provides the possibility of illuminating the samples at different spectral ranges.

In the examples above, the cooling system for cooling the containers is formed by central cooling unit 17 and the cooling assemblies 16 of the containers 5. Alternatively, however, the cooling assemblies can also operate locally, e.g. by being formed by Peltier elements, with each cooling unit attributed to a single container. However, such a system leads to temperature inhomogeneities at the cool sides of the Peltier elements, which can give rise to climate inhomogeneities within chamber 4.

The containers 5 can also be used outside chamber 4, as individual units for illuminating samples under conditions different from the climate of chamber 4.

While there are shown and described presently preferred embodiments of the invention, it is to be distinctly understood that the invention is not limited thereto but may be otherwise variously embodied and practiced within the scope of the following claims.

## Claims

1. An incubator comprising
a chamber (4),
a climate controller (11) for controlling a climate in said chamber (4),
a plurality of containers (5) arranged in said chamber (4), wherein each container (5) encloses an interior space (7) for receiving at least one sample and comprises at least one light source (14) arranged to illuminate said interior space (7).

2. The incubator of claim 1 comprising an illumination controller (15) connected to the light sources (14) of said containers (5), wherein said illumination controller (15) is structured and adapted to individually control at least one of a duration, intensity and spectral distribution of an illumination in each of said interior spaces (7).

3. The incubator of any of the preceding claims comprising at least one cooling assembly (16) for carrying off heat from said containers (5).

4. The incubator of claim 3 comprising
a cooling unit (17) cooling a cooling fluid and
cooling ducts (33) carrying said cooling fluid between said cooling unit (17) and said containers (5).

5. The incubator of any of the claims 3 or 4, wherein each container (5) comprises at least one temperature sensor (18) and wherein said incubator comprises control loops (19) for individually controlling temperatures in said containers (5).

6. The incubator of any of the preceding claims wherein, within said chamber (4), several of said containers (5) are arranged beside each other and several of said containers (5) are arranged above each other.

7. The incubator of claim 6 further comprising a plurality of storage racks (28) arranged beside each other within said chamber (4), wherein each storage rack (28) holds several containers (5) above each other.

8. The incubator of any of the preceding claims comprising a handling device (6) arranged in said chamber (4) and an automatic door (10) located in a wall of said chamber (4), wherein said handling device (6) is adapted and structured to transport samples between said door (10) and said containers (5).

9. The incubator of claim 8 comprising at least one carousel (30) in said chamber (4), wherein a plurality of said containers (5) are arranged on said carousel (30), and wherein said carousel (30) can position each of said containers (5) into an access range of said handling device (6).

10. A container, in particular for the incubator of any of the preceding claims, for receiving at least one sample and exposing said sample to controlled illumination, said container comprising
a housing enclosing an interior space (7) for receiving said at least one sample,
an opening (9) arranged in a wall of said housing, and
at least one light source (14) arranged to illuminate said interior space (7).

11. The container of claim 10, wherein said at least one light source (14) is mounted to a cooling assembly (16),
and in particular wherein said cooling assembly (16) comprises a channel (48) for a cooling fluid and an adapter (49) for connecting said channel (48) to an external source of cooling fluid.

12. The container of any of the claims 10 or 11, wherein said opening (9) covers an area of at most 25% of an area of an interior side of said wall.

13. The container of any of the claims 10 to 12, wherein said interior space (7) and said opening (9) are adapted to horizontally receive a sample holding plate of a length of 127.76 ± 0.5 mm, a width of 85.48 ± 0.5 mm and a height of 14.35 ± 0.76 mm.

14. The container of claim 13, wherein said interior space (7) has a horizontal width (W) and length (L) between 140 and 170 mm and between 100 and 130 mm, respectively, and/or wherein said opening (9) has a width not exceeding 140 mm and a height not exceeding 30 mm.

15. The container of any of the claims 10 to 14 with a sample plate (8) arranged in said interior space (7), wherein said at least one light source (14) is arranged above said sample plate (8), in particular at a distance (D) of at least 20 mm above said sample plate (8).

16. The container of any of the claims 10 to 15 comprising a plurality of light sources (14), in particular arranged in a repeating pattern.

17. The container of claim 16 comprising different light sources (14) having peak emission wavelengths differing by at least 100 nm.

18. The container of any of the claims 10 to 17, wherein a total reflectivity of interior walls of said container facing said interior space (7) is at least 80° over a spectral range of said at least one light source (14).

19. The incubator of any of the preceding claims 1 to 9 wherein said containers (5) are containers of any of the claims 10 to 18.
